# EUROPEAN PATENT APPLICATION

(11) **EP 1 388 351 A1**
(43) Date of publication of application: **11.02.2004**
(21) Application number: 02291994.8
(22) Date of filing: 08.08.2002
(51) Int. Cl.: A61P 3/04, A61K 31/216, A61K 31/4439

(54) **Use of fibrate to treat weight gain associated with rosiglitazone treatment**

(71) Applicant: LABORATOIRES FOURNIER S.A., 21000 Dijon (FR)
(72) Inventor: Junien, Jean Louis, 92310 Sevres (FR); Edgar, Alan, 21490 Saint Julien (FR); Chaput, Evelyne, 21000 Dijon (FR)
(74) Representative: Nevant, Marc

(57) **Abstract**

The present invention relates to the use of a fibrate to treat patients suffering from weight gain associated with rosiglitazone treatment.

## Description

The present invention relates to the use of a fibrate to treat patients suffering from weight gain associated with rosiglitazone treatment.

Great advances have been made in the management of diabetes during the past decade. Whereas only one class of oral medications (the sulfonylureas) was available for the treatment of type 2 diabetes in the early 1990s, new classes of oral antidiabetic agents were developed. The thiazolidinedione class of medications was first introduced to the United States when troglitazone was marketed during early 1997. Rosiglitazone, approved by the FDA during the spring of 1999, was the second thiazolidinedione to be marketed in the United States. Similar to troglitazone, rosiglitazone improves insulin sensitivity in patients with Non-Insulin-Dependent Diabetes Mellitus by activating peroxisome proliferator-activated receptor-gamma (PPARgamma) receptors in adipose tissues, skeletal muscles, and the liver.

Non-Insulin-Dependent Diabetes Mellitus (NIDDM) is a form of diabetes where utilization of insulin is not the first line therapy. It occurs predominantly in adults, in whom production of insulin is available for use, yet a defect exists in insulin-mediated utilization and metabolism of glucose and peripheral tissues. For some people with diabetes, a mutation in the genes coding for insulin, for insulin receptor and/or for insulin-mediated signal transduction factors leads to ineffective insulin and/or insulin-mediated effects, impairing the utilization or metabolism of glucose.

The pathophysiology of non-insulin-dependent diabetes mellitus consists of three major components, (1) peripheral insulin resistance; (2) increased hepatic glucose production; and (3) impaired insulin secretion. Intense research has been devoted to each of these areas, independently, in order to determine which abnormality is primary and which are secondary.

When focussing on peripheral insulin resistance, the drug of choice is a thiazolidinedione, which is a type of insulin-sensitizing agent.

The thiazolidinedione chemical series have been shown to reverse insulin resistance in patients with NIDDM and impaired glucose tolerance, and can enhance insulin action in numerous genetic and acquired rodent models of insulin resistance. The antihyperglycemic effects of thiazolidinedione result from its ability to increase insulin dependent glucose disposal and reduce hepatic glucose production. It is believed that, by enhancing insulin action, thiazolidinedione treatment results in euglycemia at a lower circulating insulin level. In this regard, studies in normal and diabetic rodents and human clinical trials have not revealed hypoglycemia as a complication of thiazolidinedione therapy. On the other hand, administration of these drugs to normal or insulin-deficient diabetic animals failed to alter plasma glucose or insulin or glucose tolerance, although insulin sensitivity was nevertheless increased.

The effects of thiazolidinediones on glucose disposal are thought to result from insulin sensitization, indicating an absolute requirement for insulin. Thiazolidinedione treatments are based on the assumption that if peripheral insulin resistance is improved, increased hepatic glucose production and impaired insulin secretion will be alleviated in due course.

It has been observed that rosiglitazone markedly increased body weight gain (E Chaput et al, Biochem Biophys Res Commun 2000 May 10;271(2):445-50). This side effect renders rosiglitazone monotherapy an undesirable prophylactic measure in the treatment of NIDDM.

As already explained, rosiglitazone is a PPARgamma activator. Other activators of peroxisome proliferator activated receptors are effective drugs to improve the metabolic abnormalities linking hypertriglyceridemia to diabetes, hyperglycemia, insulin-resistance, and atherosclerosis.

Among them, fibrates can be cited as PPARalpha activators.

Fibrates have been documented to lower plasma triglycerides and cholesterol levels and to be beneficial in the prevention of ischemic heart disease in individuals with elevated levels of LDL cholesterol. They can also modestly decrease elevated fibrinogen and PAI-1 levels. Fibrate compounds, e.g., gemfibrozil, fenofibrate, bezafibrate, and ciprofibrate, elevate the level of plasma HDL cholesterol.

The pharmacological profile of a PPARalpha activator, fenofibrate, and a PPARgamma activator, rosiglitazone, was compared (E Chaput et al, Biochem Biophys Res Commun 2000 May 10;271(2):445-50) on serum parameters, target gene expression, and body weight gain in (fa/fa) fatty Zucker rats and db/db mice as well as their association in db/db mice. Fenofibrate faithfully modified the expression of PPARalpha responsive genes. Rosiglitazone increased adipose tissue aP2 mRNA in both models while increasing liver acyl CoA oxidase mRNA in db/db mice but not in fatty Zucker rats. Both drugs lowered serum triglycerides yet rosiglitazone markedly increased body weight gain while fenofibrate decreased body weight gain in fatty Zucker rats. KRP 297, which has been reported to be a PPARalpha and gamma co-activator, also affected serum triglycerides and insulin in fatty Zucker rats although no change in body weight gain was noted. It was further observed that in db/db mice, rosiglitazone significantly increased body weight gain by 22% while the latter was non-significantly reduced by 10% by fenofibrate, and co-administration of fenofibrate and rosiglitazone did not reduce the weight gain induced by roziglitazone.

It therefore is an objective of the present invention to provide a method for treating weight gain associated with a rosiglitazone treatment.

In accomplishing this and other objectives, there has been provided, in accordance with one aspect of the present invention, a therapeutic method comprised of co-administering a pharmacologically effective dose of a fibrate, such as fenofibrate, and rosiglitazone, such that the weight gain associated with a rosiglitazone treatment is decreased.

Fibrates can be used, in combination with rosiglitazone, to treat the weight gain associated with a rosiglitazone treatment, optionally with other therapies, by improving lipidic control.

The invention includes a method of decreasing the body weight gain associated with a rosiglitazone treatment, comprising co-administering an effective dosage of a fibrate and rosiglitazone. The fibrate used in this method may be selected from the group consisting of gemfibrozil, fenofibrate, bezafibrate, clofibrate and ciprofibrate.

In another embodiment, the invention includes a method of decreasing the weight gain associated with a rosiglitazone treatment, comprising co-administering an effective dosage of a fibrate and rosiglitazone, where the effective dosage of the fibrate is in the range of about 10 to about 3000 mg per day.

In another embodiment, the effective dosage of rosiglitazone is in the range of about 0.1 to about 100 mg per day.

In another embodiment, the fibrate and rosiglitazone are administered simultaneously, in a method of decreasing the weight gain associated with a rosiglitazone treatment, comprising co-administering an effective dosage of a fibrate and rosiglitazone.

In another embodiment of a method of decreasing the weight gain associated with a rosiglitazone treatment, the fibrate and rosiglitazone are administered sequentially.

In another embodiment, the invention includes the use of a fibrate, rosiglitazone and a pharmaceutically acceptable carrier for the manufacture of a medicament for decreasing the body weight gain associated with a rosiglitazone treatment. In another embodiment, the fibrate is selected from the group consisting of gemfibrozil, fenofibrate, bezafibrate, clofibrate and ciprofibrate.

As demonstrated in the present specification, the use of a fibrate and rosiglitazone, has led to favorably unexpected results. Studies were designed to evaluate the effects of a fibrate and rosiglitazone, as a combination therapy, on weight gain in diabetic rats. The data showed that the weight gain associated with rosiglitazone monotherapy was decreased when a fibrate was administered in conjunction with rosiglitazone. The improvement was statistically significant.

Thus, this novel method reduced the weight gain associated with a rosiglitazone treatment.

As used in this application, "co-administration" means the administration of two or more compounds to the same patient, within a time period of up to about three to about four hours. For example, co-administration encompasses (1) simultaneous administration of a first and second compound; (2) administration of a first compound, followed by administration of a second compound about 2 hours after administration of the first compound; and (3) administration of a first compound, followed by administration of a second compound about 4 hours after administration of the first compound. As described herein, the present invention encompasses co-administration of a fibrate and rosiglitazone to a patient.

In the present invention, fibrates are defined as PPAR-alpha agonists (peroxisome proliferator activated receptor alpha agonists), including fibric acid derivatives and pharmaceutically acceptable salts and esters of such fibric acid derivatives. Fibric acid derivatives lower the levels of triglyceride-rich lipoproteins, such as VLDL, raise HDL levels, and have variable effects on LDL levels. The effects on VLDL levels appear to result primarily from an increase in lipoprotein lipase activity, especially in muscle. This leads to enhanced hydrolysis of VLDL triglyceride content and an enhanced VLDL catabolism. Fibric acid agents also may alter the composition of the VLDL, for example, by decreasing hepatic production of apoC-III, an inhibitor of lipoprotein lipase activity. These compounds are also reported to decrease hepatic VLDL triglyceride synthesis, possibly by inhibiting fatty acid synthesis and by promoting fatty acid oxidation.

Fibrate compounds include, but are not limited to, gemfibrozil, fenofibrate, bezafibrate, clofibrate, ciprofibrate, and analogs, derivatives and pharmaceutically acceptable salts thereof.

Fenofibrate is commercially available as TricorTM capsules. Each capsule contains 67 mg of micronized fenofibrate.

Fenofibric acid, the active metabolite of fenofibrate, lowers plasma triglycerides apparently by inhibiting triglyceride synthesis, resulting in a reduction of VLDL released into the circulation, and also by stimulating the catabolism of triglyceride rich lipoproteins (i.e. VLDL).

Clofibrate is commercially available as Atromid-S capsules. Each capsule contains 500 mg of clofibrate. Clofibrate lowers elevated serum lipids by reducing the very low-density lipoprotein fraction rich in triglycerides. Serum cholesterol may be decreased. It may inhibit the hepatic release of lipoproteins (particularly VLDL) and potentiate the action of lipoprotein lipase. The recommended daily dose of clofibrate is 2 g, administered in divided doses.

Gemfibrozil is commercially available as Lopid tablets. Each tablet contains 600 mg of gemfibrozil. Gemfibrozil is a lipid regulating agent that decreases serum triglycerides and very low density lipoprotein cholesterol, and increases high density lipoprotein cholesterol. The recommended daily dose of gemfibrozil is 1200 mg, administered in two divided doses.

Fibrates include PPAR-alpha agonists; the PPAR-alpha agonists may be identified according to an assay described in US Patent 6,008,239. Pharmaceutically acceptable salts and esters of PPAR-agonists are likewise included within the scope of this invention. Compounds which are PPAR agonists include compounds such as those described in US Patent 6,008,239, WO 97/27847, WO 97/27857, WO 97/28115, WO 97/28137 and WO 97/28149. Certain fibrate compounds as described in WO 92/10468 and WO 01/80852 are also incorporated by reference herein.

According to the present invention, the preferred fibrate is fenofibrate.

Rosiglitazone in the present invention is defined as a compound of the class of thiazolidinediones: a class of compounds which work by enhancing insulin action and promoting glucose utilization in peripheral tissue. Thiazolidinediones have no effect on insulin secretion. They apparently work by enhancing insulin action and thus promoting glucose utilization in peripheral tissues, possibly by stimulating non-oxidative glucose metabolism in muscle, and suppressing gluconeogenesis in the liver.

Rosiglitazone maleate is sold under the trademark *Avandia™* and is used in the management of type 2 diabetes mellitus (also known as non-insulin-dependent diabetes mellitus (NIDDM) or adult-onset diabetes).

Chemically, rosiglitazone maleate is (±)-5-[[4-[2-(methyl-2-pyridinylamino)ethoxy]phenyl]methyl]-2,4-thiazolidinedione, (Z)-2-butenedioate (1:1). The molecular formula is C₁₈H₁₉N₃O₃S C₄H₄O₄. The molecule has a single chiral center and is present as a racemate. Due to rapid interconversion, the enantiomers are functionally indistinguishable.

Rosiglitazone is described in US Patent 5,002,953, incorporated by reference herein.

According to the present invention, a preparation is defined as : the formulation of the active compound with encapsulating material as a carrier providing a capsule in which the active component with or without other carriers, is surrounded by a carrier, which is thus in association with it. This includes tablets, powders, capsules, pills, cachets, and lozenges which can be used as solid dosage forms suitable for oral administration.

An effective dosage is defined in the present invention as the amount of a compound that prevents or ameliorates adverse conditions or symptoms of disease(s) or disorder(s) being treated. With respect to rosiglitazone, effective dosage means a pharmacological dose in the range of about 0.1 mg/day to about 100 mg/day. A preferred dosage range is about 5 mg/day to about 50 mg/day. With respect to fibrates, effective dosage is in the range of about 10 to about 3000 mg/day. The preferred range is about 50 to about 300 mg/day. The skilled artisan will understand and appreciate that the effective dosage of a given fibrate will vary with the potency of the fibrate.

The present invention relates to the unexpected discovery that co-administration of a fibrate and rosiglitazone exerts beneficial effects on the weight gain induced by a rosiglitazone treatment.

The invention includes a method of decreasing the body weight gain associated with a rosiglitazone treatment, comprising co-administering an effective dosage of a fibrate and rosiglitazone.

The fibrate used in this method may be selected from the group consisting of gemfibrozil, fenofibrate, bezafibrate, clofibrate and ciprofibrate; fenofibrate being the preferred fibrate.

In this method, the effective dosage of the fibrate is in the range of about 10 to about 3000 mg per day and the effective dosage of rosiglitazone is in the range of about 0.1 to about 100 mg per day.

In the method of the invention, the fibrate and rosiglitazone can be administered simultaneously, or sequentially. In a preferred embodiment of the invention, the fibrate and rosiglitazone are administered simultaneously, more preferably in one formulation containing the fibrate and rosiglitazone.

Pharmaceutical formulations of the fibrate and/or rosiglitazone molecules can be prepared according to known methods. The preferred route of administering the fibrate and rosiglitazone is mucosal administration, most preferably oral administration.

For preparing pharmaceutical compositions containing a fibrate and/or roziglitazone, pharmaceutically acceptable carriers can be either solid or liquid. Solid form preparations include powders, tablets, pills, capsules, cachets, suppositories, and dispersible granules. A solid carrier can be one or more substances which may also act as diluents, flavoring agents, binders, preservatives, tablet disintegrating agents, or an encapsulating material.

In powders, the carrier is a finely divided solid which is in a mixture with the finely divided active component. In tablets, the active component is mixed with the carrier having the necessary binding properties in suitable proportions and compacted in the shape and size desired. The powders and tablets preferably contain from five or ten to about seventy percent of the active compound. Suitable carriers are magnesium carbonate, magnesium stearate, talc, sugar, pectin, dextrin, starch, gelatin, tragacanth, methylcellulose, sodium carboxymethylcellulose, a low melting wax, cocoa butter, and the like.

Liquid form preparations include solutions, suspensions, and emulsions, for example, water or water propylene glycol solutions. For parenteral injection liquid preparations can be formulated in solution e.g. in aqueous polyethylene glycol solution.

Aqueous solutions suitable for oral use can be prepared by dissolving the active component in water and adding suitable colorants, flavors, stabilizing and thickening agents as desired. Aqueous suspensions suitable for oral use can be made by dispersing the finely divided active component in water with viscous material, such as natural or synthetic gums, resins, methylcellulose, sodium carboxymethylcellulose, and other well-known suspending agents.

Also included are solid form preparations which are intended to be converted, shortly before use, to liquid form preparations for oral administration. Such liquid forms include solutions, suspensions, and emulsions. These preparations may contain, in addition to the active component, colorants, flavors, stabilizers, buffers, artificial and natural sweeteners, dispersants, thickeners, solubilizing agents, and the like.

The pharmaceutical preparation is preferably in unit dosage form. In such form the preparation is subdivided into unit doses containing appropriate quantities of the active component. The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form.

A further embodiment of the invention is related to the use of a fibrate, or a fibrate and rosiglitazone, and a pharmaceutically acceptable carrier for the manufacture of a medicament for decreasing the body weight gain associated with a rosiglitazone treatment. Such a use is especially valuable in the treatment of type 2 diabetes mellitus.

The medicament can be the pharmaceutical preparation as defined above; the fibrate is selected from the group consisting of gemfibrozil, fenofibrate, bezafibrate, clofibrate, ciprofibrate; and fenofibrate is preferred.

The invention is further illustrated by the following example, which is not to be construed as limiting, but merely as an illustration of some preferred features of the invention.

### EXAMPLE: Effect of a fibrate and rosiglitazone co-administration on body weight

This study was designed to evaluate the effects of using a combination of fibrate and rosiglitazone on body weight and in addition, if this combination therapy would prevent the body weight gain that is associated with a rosiglitazone treatment.

The data from this study, which are summarized in Table 1, demonstrate that (1) the body weight gain associated with the administration of fenofibrate is not significant, (2) body weight gain is seen upon administration of rosiglitazone, and (3) a reduction of the body weight gain associated with the administration of rosiglitazone is seen upon co-administration of fenofibrate.

Therefore, the body weight gain associated with rosiglitazone was decreased when rosiglitazone was administered in conjunction with a fibrate.

### METHOD

### Animals:

Male homozygous Zucker rats of 9 to 11 weeks of age and their lean controls were received from Iffa-Credo (France). They were put into individual cages in a temperature-, humidity- and light- controlled room (21-23°C, 12-12h light-dark cycle). They were fed with a standard laboratory diet and had free access to water. After acclimatization, they were randomized into groups of 8, based on body weight.
The experimental groups were:
- lean rats, untreated;
- obese rats, treated with the vehicle p.o., twice daily (at 8 a.m. and 8 p.m.);
- obese rats, treated with Fenofibrate, 100 mg/kg, p.o., twice daily (at 8 a.m. and 8 p.m.);
- obese rats, treated with Rosiglitazone, 1 mg/kg, p.o., twice daily (at 8 a.m. and 8 p.m.);
- obese rats, treated with Fenofibrate, 100 mg/kg and Rosiglitazone, 1 mg/kg, p.o., twice daily (at 8 a.m. and 8 p.m.).
*Statistics :* All data are presented as mean ± sem. They were subjected to ANOVA followed by Student-Newman-Keuls test. A p < 0.05 was considered significant.

**Table 1**

| | **Treatment** | **Initial body weight (g)** | **Final body weight (g)** | **Body weight gain (over 15 days) (g)** |
|---|---|---|---|---|
| **Lean FA/?** | **No treatment** | 318.6 ± 7.1 | 336.8 ±6.0 | 18.2 ± 1.9 |
| **Obese** fa/fa | **Vehicle** | 390.2 ±7.0 | 424.9 ± 7.2 | 34.7 ± 3.3 |
| | **Fenofibrate 100 mg/kg** | 413.8 ± 6.6 | 422.8 ± 6.4 | 9.0 ± 3.7⁽¹⁾ |
| | **Rosiglitazone 1 mg/kg Fenofibrate 100 mg/kg +** | 411.6 ± 7.2 | 454.6 ± 9.0 | 43.0 ± 5.4 |
| | **Rosiglitazone 1 mg/kg** | 401.7 ± 5.7 | 432.3 ± 5.4 | 30.6 ± 6.3 |

| | | | | |
|---|---|---|---|---|
| (1) not significant | | | | |

## Claims

1. A method of decreasing the body weight gain associated with a rosiglitazone treatment, comprising co-administering an effective dosage of a fibrate and roziglitazone.

2. The method according to claim 1, wherein the fibrate is selected from the group consisting of gemfibrozil, fenofibrate, bezafibrate, clofibrate and ciprofibrate.

3. The method according to claim 1 or 2, wherein the fibrate is fenofibrate.

4. The method according to one of claims 1 to 3, wherein the effective dosage of the fibrate is in the range of about 10 to about 3000 mg per day.

5. The method according to one of claims 1 to 4, wherein the effective dosage of rosiglitazone is in the range of about 0.1 to about 100 mg per day.

6. The method according to one of claims 1 to 5, wherein the fibrate and rosiglitazone are administered simultaneously.

7. The method according to one of claims 1 to 6, wherein the fibrate and rosiglitazone are administered sequentially.

8. Use of a fibrate and a pharmaceutically acceptable carrier for the manufacture of a medicament for decreasing the body weight gain associated with a rosiglitazone treatment.

9. Use of a fibrate, rosiglitazone and a pharmaceutically acceptable carrier for the manufacture of a medicament for decreasing the body weight gain associated with a rosiglitazone treatment.

10. The use according to claim 8 or 9, wherein the fibrate is selected from the group consisting of gemfibrozil, fenofibrate, bezafibrate, clofibrate, ciprofibrate.

11. The use according to one of claims 8 to 10, wherein the fibrate is fenofibrate.

12. The use according to one of claims 8 to 11, wherein the fibrate and rosiglitazone are administered simultaneously or sequentially.
